(19) Europäisches Patentamt
European Patent Office
Office européen des brevets

(11) Veröffentlichungsnummer: **0 016 867**
**B1**

(12) # EUROPÄISCHE PATENTSCHRIFT

(45) Veröffentlichungstag der Patentschrift: **22.06.83**

(21) Anmeldenummer: **79105314.3**

(22) Anmeldetag: **21.12.79**

(51) Int. Cl.³: **C 07 C 59/295,** C 07 D 305/12, C 07 C 51/09, A 61 K 31/00, C 07 B 19/00 //C07D303/38, C07D301/12

(54) **Monochlorcitronensäurederivate, diese enthaltende pharmazeutische Präparate und Verfahren zur Herstellung dieser Derivate.**

(30) Priorität: **26.12.78 US 973504**
**28.11.79 CH 10580/79**

(43) Veröffentlichungstag der Anmeldung:
**15.10.80 Patentblatt 80/21**

(45) Bekanntmachung des Hinweises auf die Patenterteilung:
**22.06.83 Patentblatt 83/25**

(84) Benannte Vertragsstaaten:
**AT BE CH DE FR GB IT LU NL SE**

(56) Entgegenhaltungen:
**US - A - 3 356 721**
**US - A - 3 764 692**
**US - A - 3 901 915**
**US - A - 4 123 458**

**CHEMICAL ABSTRACTS, Band 88, 1978, Seite 89, Zusammenfassung Nr. 84165k, Columbus, Ohio, US, R. J. JAEGER et al.: "Effect of 1,1-dichloroethylene exposure on hepatic mitochondria"**

(73) Patentinhaber: **F. HOFFMANN-LA ROCHE & CO. Aktiengesellschaft**
**CH-4002 Basel (CH)**

(72) Erfinder: **Guthrie, Robert William**
**102 Alberta Drive**
**Saddle Brook, N.J. 07662 (US)**
Erfinder: **Kierstead, Richard Wightman**
**30 Willowbrook**
**North Caldwell, N.J. 07006 (US)**
Erfinder: **Mennona, Francis A.**
**380 Bloomfield Avenue**
**Nutley, N.J. 07110 (US)**
Erfinder: **Sullivan, Ann Clare**
**48 Lakewood Avenue**
**Cedar Grove, N.J. 07009 (US)**

(74) Vertreter: **Lederer, Franz, Dr. et al,**
**Patentanwälte Dr. Franz Lederer Reiner F. Meyer**
**Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 016 867 B1

# Monochlorcitronensäurederivate, diese enthaltende pharmazeutische Präparate und Verfahren zur Herstellung dieser Derivate

Die vorliegende Erfindung betrifft neue Monochlorcitronensäurederivate der Formel

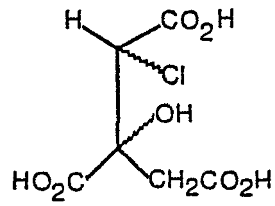

Ia

und die entsprechenden threo-β-Lactone der Formel

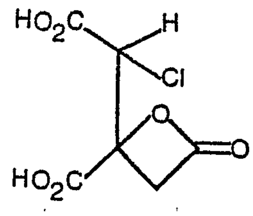

Ib

sowie die pharmazeutisch verwendbaren Salze dieser Verbindungen.

Die Monochlorcitronensäurederivate der Formel Ia und einige Ausgangsmaterialien und Zwischenprodukte in deren Herstellung enthalten zwei asymmetrische Kohlenstoffatome und liegen daher in threo- oder erythro-Form vor. Die Chlorcitronensäure-β-lactone der Formel Ib liegen in threo-Form vor. Die threo-Form und die erythro-Form können wiederum als Racemate oder als optische Antipoden, das (+)- und das (—)-Antipode, vorliegen. Im Zusammenhang mit der threo-erythro-Nomenklatur kann auf J. Amer. Chem. Soc., 74, 5828 (1952) und Experientia, 12, 81 (1956) hingewiesen werden.

Die Ausdrücke "Alkalimetall" und "Erdalkalimetall" bezeichnen Lithium, Natrium und Kalium bzw. Calcium. Der Ausdruck "Alkanol" betrifft einen einem geradkettigen oder verzweigten $C_{1-20}$—Alkan entsprechenden Alkohol. Beispiele von Alkanolen sind Methanol, Aethanol und 2—Propanol.

Die Verbindungen der Formeln Ia und Ib und die Salze davon haben anorektische Wirkung und können daher als Anorektika zur Behandlung von Fettsucht bei Säugetieren verwendet werden. Die Erfindung betrifft die Verbindungen der Formeln Ia und Ib und die pharmazeutisch verwendbaren Salze davon als pharmazeutischen, insbesondere anorektische, Mittel sowie Arzneimittel, insbesondere solche mit anorektischer Wirkung, enthaltend eine Verbindung der Formel Ia oder Ib oder ein pharmazeutisch verwendbares Salz davon und Verfahren zur Herstellung solcher Arzneimittel.

Die Erfindung betrifft ferner ein Verfahren zur Herstellung der Verbindungen der Formeln Ia und Ib und der pharmazeutisch verwendbaren Salzen davon. Dieses Verfahren ist dadurch gekennzeichnet, dass man

a) zur Herstellung eines (±)-threo-Lactons der Formel Ib, eine wässrige Lösung eines Trialkalimetall- oder Trierdalkalimetall-cis- oder -trans-aconitats mit Chlor oder unterchloriger Säure umsetzt und das erhaltene Salz des (±)-threo-chlorcitronensäure-β-lactons der Formel

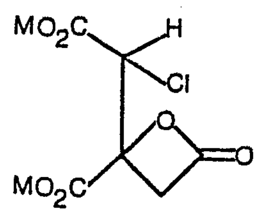

Ib 1

worin M ein Alkali- oder Erdalkalimetall ist, mit einer Säure umsetzt,

b) zur Herstellung der (±)-threo-Chlorcitronensäure der Formel

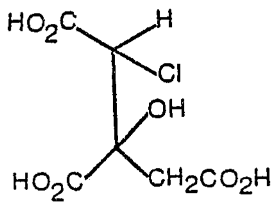

Ia 1

eine Verbindung der Formel Ib oder Ib 1 hydrolysiert,
c) zur Herstellung einer Verbindung der Formel Ia Epoxyaconitsäure mit einem Alkali- oder Erdalkalimetallchlorid in einem wässrigen Lösungsmittel in Gegenwart einer Säure spaltet,
d) zur Herstellung von (±)-erythro-Chlorcitronensäure der Formel

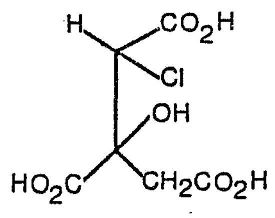

Ia 2

ein Epoxyd der Formel

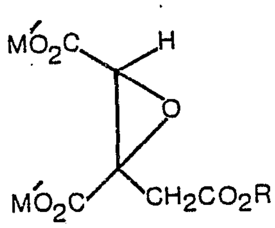

II

worin M′ ein Alkalimetall und R Wasserstoff oder M′ darstellt,
mit einem Alkalimetallchlorid in einem wässrigen Lösungsmittel in Gegenwart einer Säure spaltet,
e) gewünschtenfalls ein erhaltenes (±)-threo-Citronensäurederivat der Formel Ia oder Ib oder (±)-erythro-Citronensäurederivat der Formel Ia in die optisch aktiven Antipoden auftrennt und das erwünschte Antipode isoliert,
f) gewünschtenfalls eine erhaltene Verbindung der Formel Ia oder Ib in Form eines pharmazeutisch verwendbaren Salzes isoliert.

Die wässrige Lösung von Verfahrensstufe a), die durch Auflösung von Aconitsäure in einer wässrigen Lösung eines Alkali- oder Erdalkalimetallhydroxyds, vorzugsweise Natrium- oder Kaliumhydroxyd erhalten werden kann, wird zweckmässigerweise auf etwa 0 bis 30°C, vorzugsweise 5°C, abgekühlt und mit einem Ueberschuss an Chlor oder unterchloriger Säure, vorzugsweise Chlor, behandelt.

Als Lösungsmittel werden zweckmässigerweise Wasser oder Gemische von Wasser und einem nieder-Alkanol, von Wasser und einem Aether, z.B. Dimethoxyäthan, Tetrahydrofuran oder Dioxan, oder von Wasser und einem polaren aprotischen Lösungsmittel, wie Dimethylacetamid, Dimethylformamid, Dimethylsulfoxyd oder Hexamethylphosphoramid verwendet.

Als Säure, die zweckmässigerweise zur Ueberführung des Salzes Ib 1 in die entsprechende freie Säure Ib verwendet werden können, sind Mineralsäuren, wie Salzsäure, Schwefelsäure, Salpetersäure oder Phosphorsäure, Sulfonsäuren, wie Methan-, Phenyl- oder p-Toluolsulfonsäure, und starke organische Carbonsäuren, wie Trifluor- oder Trichloressigsäure, zu nennen.

Die Hydrolyse der β-Lactonfunktion einer Dicarbonsäure der Formel Ib oder des Disalzes davon der Formel Ib 1 nach Verfahrensstufe b) kann dadurch bewerkstelligt werden, dass man die Dicarbonsäure oder das Disalz in einem eine der weiter oben genannten Säure enthaltenden wässrigen Lösungsmittel suspendiert oder löst und zur Vervollständigung der Hydrolyse zweckmässigerweise bei einer Temperatur von etwa 30—80°C erhitzt. Eine Temperatur von etwa 50—70°C, insbesondere etwa 70°C, ist bevorzugt.

Die Anlagerung von unterchloriger Säure der Stufe a) und die Hydrolyse der Stufe b) können stufenweise durchgeführt werden. Zweckmässigerweise wird jedoch das Disalz Ib 1 angesäuert und das erhaltene Reaktionsgemisch zur Vervollständigung der Hydrolyse des Disalzes Ib zur (±)-threo-Chlorcitronensäure erhitzt. So wird nach Vervollständigung der Anlagerung von unterchloriger Säure das Reaktionsgemisch vorzugsweise mit einer Mineralsäure, zweckmässigerweise Salzsäure, angesäuert und zur Vervollständigung der Ueberführung des β-Lactons Ib zur Säure Ia 1 bei einer Tempera-

tur von etwa 30—100°C, vorzugsweise etwa 50—90°C, zweckmässigerweise etwa 70°C, erhitzt.

Die Spaltung von Stufe c) wird mit einem Alkali- oder Erdalkalimetallchlorid in einem Lösungsmittel in Gegenwart einer Säure durchgeführt.

So kann man (±)-threo-Epoxyaconitsäure mit einem Alkalimetallchlorid in einem wässrigen Lösungsmittel in Gegenwart einer Säure, vorzugsweise mit einem Ueberschuss an Natriumchlorid in Wasser in Gegenwart eines Mol-äquivalentes Salzsäure, bei einer Temperatur zwischen etwa 50 und 80°C, vorzugsweise etwa 70°C, spalten.

In analoger Weise wird (+)-threo-Epoxyaconitsäure zu (—)-threo-Chlorcitronensäure, (—)-threo-Epoxyaconitsäure zu (+)-threo-Chlorcitronensäure, (—)-erythro-Epoxyaconitsäure zu (+)-erythro-Chlorcitronensäure und (+)-erythro-Epoxyaconitsäure zu (—)-erythro-Chlorcitronensäure mittels eines Alkalimetallchlorids in einem wässrigen Lösungsmittel in Gegenwart einer Säure, vorzugsweise einem Ueberschuss an Natriumchlorid in Wasser in Gegenwart eines Mol-äquivalentes Salzsäure, bei einer Temperatur zwischen etwa 50 und 80°C, vorzugsweise 70°C, gespalten.

(±)-erythro-Chlorcitronensäure kann in hoher Ausbeute ausgehend von (±)-erythro-Epoxyaconitsäure erhalten werden, wobei letztere durch Epoxydierung von cis-Aconitsäure oder deren Anhydrid in situ hergestellt werden kann. Die Epoxydierung wird zweckmässigerweise mittels Wasserstoffperoxyd und einem Epoxydierungskatalysator wie Wolframsäure oder einem Salz davon, vorzugsweise einem Alkalimetallsalz, zweckmässigerweise dem Natriumsalz, durchgeführt. Die Epoxydierung wird vorzugsweise in Wasser enthaltend von etwa 0 bis 2,9 Mol-äquivalente eines Alkalimetallhydroxyds, vorzugsweise etwa 2,5 Mol-äquivalente Natriumhydroxyd, durchgeführt. Anstelle von Wasser kann man ein organisches Lösungsmittel, wie ein nieder-Alkanol oder einen wasserlöslichen Aether, wie Dimethoxyäthan, Tetrahydrofuran oder Dioxan, verwenden. Die Epoxydierung wird zweckmässigerweise bei einer Temperatur zwischen etwa 0 und 100°C, vorzugsweise von etwa 20—50°C, durchgeführt. Die entstandene (±)-erythro-Epoxyaconitsäure oder das Salz davon der Formel II kann ohne Isolierung angesäuert und dann mittels eines Alkalimetallchlorids, vorzugsweise Natriumchlorid, gespalten werden. Als Säure können Mineralsäuren, wie Salzsäure, Schwefelsäure oder Phosphorsäure, Sulfonsäuren, z.b. Methan-, Phenyl- oder p-Toluolsulfonsäure, und starke organische Säuren, z.B. Trifluor- oder Trichloressigsäure, verwendet werden. Die Salzsäure ist bevorzugt.

Um Nebenreaktionen der erhaltenen (±)-erythro-Chlor-citronensäure zu vermeiden wird die Spaltung vorzugsweise in Gegenwart von etwa 1—10 Mol-äquivalenten der weiter oben erwähnten Säuren durchgeführt. Wenn die Epoxydierung in Abwesenheit eines Alkalimetallhydroxyds durchgeführt wird, werden etwa 1—10 Mol-äquivalente Säure verwendet und wenn die Epoxydierung in Gegenwart von etwa 2,5 Mol-äquivalenten Alkalimetallhydroxyd durchgeführt wird, werden etwa 3,5—12,5 Mol-äquivalente Säure verwendet.

Die Reaktionstemperatur ist nicht kritisch. Jedoch wird die Spaltung vorzugsweise bei einer Temperatur zwischen etwa 50 und 80°C, vorzugsweise etwa 70°C, durchgeführt.

Während man bei den weiter oben beschriebenen Verfahren zur Herstellung von (±)-erythro-Chlorcitronensäure Ia 2 von in situ hergestellter (±)-erythro-Epoxyaconitsäure ausgeht, kann man auch diese Säure wie in dem U.S. Patent 3,969,772 herstellen und isolieren und dann zur (±)-erythro-Chlorcitronensäure spalten, wobei die Spaltung in der gleichen Weise wie für (±)-threo-Epoxyaconitsäure durchgeführt wird.

Während die optisch aktiven Citronensäurederivate der Formeln Ia und Ib zweckmässigerweise ausgehend von den optischen Antipoden der Epoxyaconitsäure hergestellt werden, kann man auch diese Verbindungen durch an sich bekannte Auftrennungsmethoden ausgehend von racemischen threo- und erythro-Citronensäurederivaten herstellen.

So kann z.B. (±)-erythro-Chlorcitronensäure mittels (+)- und (—)-p-Nitro-$\alpha$-methylbenzylamin nach der in der U.S. Patentschrift 3,901,915 beschriebenen Methode in ihre optischen Antipoden (+)- und (—)-erythro-Chlorcitronensäure aufgetrennt werden.

Das (±)-threo-Chlorcitronensäure-$\beta$-lacton kann nach den weiter oben beschriebenen Methoden in seine optischen Antipoden aufgetrennt werden. Insbesondere kann das (±)-threo-Chlorcitronensäure-$\beta$-lacton mit (+)-p-Nitro-$\alpha$-methylbenzylamin in einem nieder-Alkanol, wie Methanol, zu den diastereomeren Salzen von threo-Chlorcitronensäure-$\beta$-lacton umgesetzt werden und die erhaltenen Salze können in an sich bekannter Weise, z.B. durch Kristallisation, aufgetrennt werden.

Eine vorteilhafte Methode zur Herstellung von (±)-threo-Chlorcitronensäure Ia 1 besteht darin, dass man trans-Aconitsäure zu einem leicht isolierbaren hochkristallinen Monoalkalimetallsalz von (±)-threo-Epoxyaconitsäure umwandelt und dieses wie weiter oben beschrieben, z.B. mit Natriumchlorid in Gegenwart von Salzsäure, spaltet.

Die Ueberführung von trans-Aconitsäure zum Monoalkalimetallsalz der (±)-threo-Epoxyaconitsäure kann durch verschiedene Verfahren bewerkstelligt werden.

In einem ersten Verfahren wird die trans-Aconitsäure nach der weiter oben beschriebenen Methode in ein Dialkalimetallsalz des (±)-threo-Chlorcitronensäure-$\beta$-lactons übergeführt. Anstatt das $\beta$-Lacton wie oben beschrieben direkt zur Chlorcarbonsäure Ia 1 unter sauren Bedingungen zu hydrolysieren, wird zuerst die $\beta$-Lactonfunktion hydrolysiert und gleichzeitig die Chlorfunktion unter alkalischen Bedingungen zu einem Trialkalimetallsalz der (±)-threo-Epoxyakonitsäure verschoben und dann das Trisalz durch Partialhydrolyse in das erwünschte Monosalz übergeführt.

Die alkalisch induzierte Hydrolyse-Verschiebung des Dialkalimetallsalzes der (±)-threo-Chlorcitronensäure-β-lactons wird mit einem Alkalimetallhydroxyd, vorzugsweise Kaliumhydroxyd, bei einer Temperatur zwischen etwa 0 und 40°C, vorzugsweise etwa 0—25°C, durchgeführt.

Die Partialneutralisierung des Trisalzes der (±)-threo-Epoxyaconitsäure wird durch Einstellung des pH—Wertes des Reaktionsgemisches auf etwa 7,0—7,5, vorzugsweise etwa 7,2, Abkühlen des Reaktionsgemisches auf etwa —20 bis 20°C, vorzugsweise etwa 0—5°C, Zusatz von etwa 2 Mol-äquivalenten Säure und Reinigung des erhaltenen Niederschlags durch Umkristallisation aus Wasser oder Wasser-Alkanol-Gemischen, vorzugsweise Wasser, durchgeführt.

In dem zweiten Verfahren wird trans-Aconitsäure durch Anlagerung von unterchloriger Säure in ein Trialkalimetallsalz der (±)-threo-Chlorcitronensäure übergeführt und diese dann durch Cyclisierung unter alkalischen Bedingungen und Partialneutralisation unter sauren Bedingungen in ein Trialkalimetallsalz der (±)-threo-Epoxyaconitsäure übergeführt.

Die Anlagerung von unterchloriger Säure wird dadurch bewerkstelligt, dass man trans-Akonitsäure mit einem Alkalimetallhypochlorit, vorzugsweise Kaliumhypochlorit, behandelt, wobei das Hypochlorit durch Lösung von Chlor in einer Alkalimetallhydroxydlösung, vorzugsweise wässrigem Kaliumhydroxyd, hergestellt wurde. Die Temperatur ist nicht kritisch, jedoch wird vorzugsweise bei einer Temperatur zwischen etwa —20 und 25°C, zweckmässigerweise etwa —5 bis 5°C, insbesondere bei etwa 0°C, gearbeitet. Es werden etwa 2 Mol-äquivalente Alkalimetallhydroxyd zur Bildung eines Dialkalimetallsalzes der trans-Aconitsäure und dan zwei weitere Mol-äquivalente Alkalimetallhydroxyd zur Bildung des für die Anlagerung von unterchloriger Säure an das Disalz notwendigen Alkalimetallhypochlorits benötigt.

Die Cyclisierung des Trialkalimetallsalzes der (±)-threo-Chlorcitronensäure zu dem Trialkalimetallsalz der (±)-threo-Epoxyaconitsäure wird dadurch bewerkstelligt, dass man das Reaktionsgemisch oder das Trialkalimetallsalz der (±)-threo-Chlorcitronensäure in einem Lösungsmittel mit einem Alkalimetallhydroxyd, vorzugsweise Kaliumhydroxyd, behandelt. Die Cyclisierungstemperatur ist nicht kritisch, jedoch wird vorzugsweise bei einer Temperatur zwischen etwa 15 und 60°C, vorzugsweise etwa 25°C, gearbeitet. Die Cyclisierung wird zweckmässigerweise in einem Lösungsmittel, wie Wasser oder einem Gemisch von Wasser und einem nieder-Alkanol, vorzugsweise Wasser, durchgeführt.

Die Partialneutralisierung des Trialkalimetallsalzes der (±)-threo-Epoxyaconitsäure wird dadurch bewerkstelligt, dass man es oder das Reaktionsgemisch, in dem es gebildet wurde, mit einer Mineralsäure oder einer organischen Säure in zu der weiter oben beschriebenen Umsetzung des Dialkalimetallsalzes des (±)-threo-Chlorcitronensäure-β-lactons analoger Weise behandelt.

In einem dritten Verfahren, einer Variante des zweiten, werden etwa zweidrittel eines Mol-äquivalentes trans-Aconitsäure mit etwa 2 Mol-äquivalenten eines Alkalimetallhydroxyds, vorzugsweise Kaliumhydroxyd, in einem Lösungsmittel, dann mit etwa einem Mol-äquivalent Alkalimetallhypochlorit, vorzugsweise Kaliumhypochlorit, und etwa eindrittel Mol-äquivalent trans-Aconitsäure zu einem Trialkalimetallsalz der (±)-threo-Chlorcitronensäure umgesetzt.

Als Lösungsmittel können Wasser und Gemische von Wasser und nieder-Alkanolen, vorzugsweise Wasser, verwendet werden. Die Temperatur ist nicht kritisch. Man arbeitet jedoch vorzugsweise bei einer Temperatur zwischen etwa —20 und 10°C, insbesondere von etwa —10 bis —5°C.

Das so erhaltene Trisalz wird in der für das erste Verfahren beschriebenen Weise in das Monoalkalimetallsalz der (±)-threo-Epoxyaconitsäure übergeführt.

In dem vierten Verfahren wird (±)-threo-Epoxyaconitsäure mit etwa einem Aequivalent eines Alkalimetallhydroxyds, vorzugsweise Kaliumhydroxyd, in einem Lösungsmittel, wie Wasser oder einem Gemisch von Wasser und eines nieder-Alkanols, in ein Monoalkalimetallsalz, vorzugsweise das Kaliumsalz, übergeführt. Die Temperatur ist nicht kritisch. Man arbeitet jedoch vorzugsweise bei einer Temperatur von etwa 5°C. Das erhaltene Monosalz wird in der gleichen Weise wie für die anderen Varianten isoliert und gereinigt. Die Monoalkalimetallsalze der (±)-Epoxyaconitsäure werden gewöhnlich als Monohydrate isoliert.

Die (—)-threo-Chlorcitronensäure kann dadurch hergestellt werden, dass man ein Monoalkalimetallsalz der (±)-threo-Epoxyaconitsäure zu (±)-threo-Epoxyaconitsäure neutralisiert, diese dann mit (+)-p-Nitro-α-methylbenzylamin zu (+)-threo-Epoxyaconitsäure auftrennt und diese nach den weiter oben beschriebenen Methoden zu (—)-threo-Chlorcitronensäure spaltet.

Die Neutralisierung wird zweckmässigerweise durch Behandlung des Monoalkalimetallsalzes, vorzugsweise des Kaliumsalzes, mit einer starken Säure in einem Lösungsmittel durchgeführt. Als Säure können Mineralsäure wie Salzsäure, Bromwasserstoffsäure, Salpetersäure, Perchlorsäure und Schwefelsäure, und organische Säure wie Methan-, Benzol-, p-Toluolsulfonsäure, Trifluor- und Trichloressigsäure, verwendet werden. Als Lösungsmittel können Wasser, nieder-Alkanole, Gemische von Wasser und nieder-Alkanolen und Ketone, wie Aceton, Methyläthylketon und Diäthylketon, verwendet werden. Mineralsäuren und Ketone, insbesondere Schwefelsäure und Aceton, sind bevorzugt.

Von besonderem Interesse Als Anorektika sind die Verbindungen der Formel Ia und die pharmazeutisch verwendbaren Salze davon, insbesondere (—)-threo-Chlorcitronensäure und die pharmazeutisch verwendbaren Salzen davon, dies auf Grund ihrer im Vergleich zu Hydroxycitronensäure und Citronensäure besseren Fähigkeit den Futterverbrauch zu erniedrigen.

Weiblichen Ratten mit einem Gewicht von 150—175 g in individuellen Käfigen wird während 48 Stunden keine Nahrung gegeben. Dann werden die Tiere 5—13 Tage 3 Stunden täglich mit einem 70% Glucose enthaltendem Futter gefüttert. Danach wird en Tieren eine halbe Stunde vor dem 3-stündigen Füttern die Testsubstanz in wässriger Lösung oral verabreicht. Nach dem Füttern werden die Futterschalen gewogen. Die Kontrollgruppe besteht aus 31 Tieren. Jede der mit einer Testsubstanz behandelten Gruppe besteht aus 5—12 Ratten. Die Resultate sind in Tabelle I angegeben.

TABELLE I

| Konzentration mMol/kg Körpergewicht | Futterverbrauch | | | |
|---|---|---|---|---|
| | (±)-threo-Chlorcitronensäure | | Trinatrium (—)-threo-hydroxycitrat | |
| | g[a] | % der Kontrolle | g[a] | % der Kontrolle |
| Kontrolle | 11,2±0,5 | 100 | 11,2±0,5 | 100 |
| 2,63 | 3,8±0,3* | 34 | 8,9±0,8* | 79 |
| 1,32 | 3,1±0,4* | 28 | 9,9±1,0 | 88 |
| 0,66 | 3,9±0,3* | 35 | 9,0±0,6* | 80 |
| 0,33 | 5,7±0,5* | 51 | 11,5±0,7 | 103 |
| 0,17 | 6,8±0,7* | 61 | | |
| 0,08 | 6,4±0,6* | 57 | | |

[a]Mittelwert *$p \leq 0,01$

Weiblichen Ratten mit einem Gewicht von 130—150 g in individuellen Käfigen wird während 48 Stunden kein Futter gegeben. Dann werden die Tiere 5—12 Tage 3 Stunden täglich mit einem 70% Glucose enthaltendem Futter gefüttert. Die Testsubstanzen werden den Tieren eine halbe Stunde vor dem Füttern oral verabreicht. Die Futterschalen werden sofort nach dem Füttern gewogen. Die Kontrollgruppe besteht aus 5—10 Tieren und die Testgruppen aus 4—6 Tieren. Die Resultate sind in Tabelle II angegeben.

TABELLE II

| Behandlung | Futterverbrauch | | | |
|---|---|---|---|---|
| | 2,63 mMol/kg Körpergewicht | | 0,66 mMol/kg Körpergewicht | |
| | g[a] | % der Kontrolle | g[a] | % der Kontrolle |
| Kontrolle | 13,5±0,8 | 100 | 8,2±1,2 | 100 |
| Citronensäure | 12,7±1,7 | 94 | — | — |
| (+)-threo-Citronensäure | 5,1±1,2** | 38 | 6,8±1,0 | 83 |
| (—)-threo-Chlorcitronensäure | 2,2±0,5** | 16 | 4,8±0,7* | 59 |
| (—)-erythro-Chlorcitronensäure | 7,2±1,5** | 53 | 7,0±1,9 | 85 |
| (+)-erythro-Chlorcitronensäure | 10,5±0,8* | 78 | 8,1±1,6 | 99 |

[a]Mittelwert

* $p \leq 0,05$ ** $p \leq 0,001$

Die Citronensäurederivate der vorliegenden Erfindung können in Form pharmazeutischer Präparate Verwendung finden, welche sie mit einem für die enterale oder parenterale Applikation geeigneten organischen oder anorganischen Trägermaterial, wie Wasser, Gelatine, Lactose, Stärke, Magnesiumstearat, Talk oder pflanzliche Oele enthalten. Die pharmazeutischen Präparate können in konventioneller Form vorliegen, z.B. in fester Form, z.B. als Tabletten, Dragèes, Kapseln oder Suppositorien oder in flüssiger Form, wie als Suspensionen oder Emulsionen. Sie können auch in konventioneller Form behandelt, z.B. sterilisiert, werden und konventionelle pharmazeutische Hilfsstoffe, wie Konservierungs-, Stabilisierungs- oder Emulgiermittel, Salze zur Veränderung des osmotischen Druckes oder Puffer enthalten. Sie können auch noch andere therapeutisch wertvolle Stoffe enthalten.

Eine pharmazeutische Dosierungseinheit kann von etwa 10—1000 mg (—)-threo-Chlorcitronensäure oder eines ihrer Isomeren enthalten. Die tägliche parenterale und orale Verabreichungsdosis beträgt bei Säugetieren von etwa 1 bis 150 mg/kg.

Die Citronensäurederivate der vorliegenden Erfindung können auch Tierfutterzusätzen, -vormischungen oder -konzentrate beigemischt werden.

Der Aktivsubstanzgehalt der Futtervormischungen oder des kompletten Futters kann von etwa 0,0025 bis 1%, vorzugsweise von etwa 0,0625 bis 0,40%, insbesondere etwa 0,125% der täglichen Futtereinnahme betragen.

In Res. Commun. in Chem. Pathology Pharmacology, 1977, *18* (1), 83—94 (C.A. *88*, 1978, S. 89 — Nr. 84165 K) wird die Meinung vertreten, dass das 1,1-Dichloräthylen (1,1—DCE) in der Leber über die Monochloressigsäure zu einer hepatotoxischen Substanz metabolisiert wird und dass die Hepatotoxizität des 1,1—DCE auf diejenige dieser Substanz zurückzuführen ist. Die im besagten Artikel geäusserte Hypothese, dass diese hepatotoxische Substanz die Monochlorcitronensäure ist, sollte nicht als stichhaltig angesehen werden. Wie nämlich aus den weiter oben beschriebenen Versuchen hervorgeht, wurde Ratten bis zu 2,63 mMol/kg Chlorcitronensäure verabreicht. Dabei wurde lediglich eine anorektische Wirkung der Monochlorcitronensäure festgestellt.

Die US Patentschrift 3,356,721 beschreibt die Synthese der Citronensäure und von deren Derivaten über entsprechende $\beta$-Lactone der Formel A in Spalte 7, nicht aber die Herstellung der Monochlorcitronensäure.

Die US Patentschrift 4.123.458 beschreibt die Herstellung von chlorierten Tetracarbonsäurederivaten der in den Spalten 22 und 30 dargestellten Formeln, nicht aber die Herstellung der chlorierten Tricarbonsäurederivate der vorliegenden Erfindung.

Die US Patentschriften 3.901.915 und 3.764.692 beschreiben die optische Auftrennung von Citronensäurederivaten sowie die Hemmung der Fettsäuresynthese durch diese Citronensäurederivate. Letztere sind strukturell völlig verschieden von den Verbindungen der Formeln Ia und Ib.

## Beispiel 1

Herstellung des Ausgangsmaterials.

174 g trans-Aconitsäure wurden portionenweise einer gerührten Lösung von 120 g Natriumhydroxyd in 400 ml Wasser zugesetzt. Die Temperatur wurde bei 25° gehalten. Nach vollständiger Auflösung der Säure war der pH—Wert 7,5.

Verfahren.

Die Lösung wurde auf 5° abgekühlt und mit Argon gereinigt. Dann wurde gasförmiges Chlor dem gerührten Gemisch zugeleitet. Die Temperatur wurde bei 10—15° gehalten. Als kein Gas mehr absorbiert wurde, wurde die Chloreinführung abgestellt und das Gemisch 10 Minuten bei 10° gerührt. Das Gemisch wurde mit Argon von überschüssigem Chlor gereinigt.

Das Gemisch wurde mit 175 ml konzentrierter Salzsäure angesäuert und dann 1 Stunde auf 70° erhitzt, wobei das $\beta$-Lacton hydrolysiert wurde. Die Lösung wurde zur Trockene eingeengt und der Rückstand mit Aethylacetat vermischt. Die vereinigten Extrakte wurden filtriert und getrocknet. Nach Abdampfung des Lösungsmittels wurde der Feststoff in Aethylacetat gelöst. Die Lösung wurde mit Tetrachlorkohlenstoff verdünnt. Nach Rühren und Filtrieren erhielt man 102,0 g ($\pm$)-threo-Chlorcitronensäure, Schmelzpunkt 96—101°.

Die Mutterlaugen wurden zur Trockene eingeengt und dann wie oben beschrieben kristallisiert, wobei man zusätzliche 60,7 g reine ($\pm$)-threo-Chlorcitronensäure erhielt.

## Beispiel 2

Einer Lösung von 123 g mono-Kalium-($\pm$)-threo-epoxyaconit-monohydrat, und 28 g Kaliumchlorid in 120 ml Wasser wurden 88 ml konzentrierter Salzsäure zugesetzt. Das Reaktionsgemisch wurde 15 Stunden auf 70° erhitzt, dann auf Raumtemperatur abgekühlt und konzentriert. 250 ml Aethylacetat wurden zugesetzt und das Gemisch wurde bei 40° gerührt. Das ausgefallene Kaliumchlorid wurde aufgesammelt und mit 350 ml Aethylacetat gewaschen. Das Filtrat wurde zur Trockene eingedampft. Der Rückstand wurde in Aethylacetat gelöst, mit wasserfreiem Magnesiumsulfat behandelt und filtriert. Der Filterkuchen wurde mit Aethylacetat gewaschen. Tetrachlorkohlenstoff wurde dem Filtrat zugesetzt. Das Gemisch wurde mit kristallinem ($\pm$)-threo-Chlorcitronensäure-monohydrat beimpft, 2 Stunden gerührt und 16 Stunden im Kühlschrank aufbewährt. Der Niederschlag wurde aufgesammelt,

mit Tetrachlorkohlenstoff-Aethylacetat (3:1) gewaschen und zu 70,9 g (±)-threo-Chlorcitronensäure-monohydrat, Schmelzpunkt 74—76° getrocknet.

Die Mutterlaugen wurden zur Trockene eingedampft. Der Rückstand wurde in 125 ml Aethylacetat gelöst und mit Tetrachlorkohlenstoff behandelt, wobei man 20,7 g Produkt erhält.

Eine 91,0 g Portion der vereinigten ersten und zweiten Produktanteile wurde in 250 ml Aethylacetat gelöst und mit 500 ml Tetrachlorkohlenstoff behandelt. Die Lösung wurde mit kristallinem (±)-threo-Chlorcitronensäure-monohydrat beimpft und über Nacht im Kühlschrank aufbewahrt. Der Niederschlag wurde aufgesammelt, mit Tetrachlorkohlenstoff und Aethylacetat gewaschen und zu 84,3 g reinem Produkt, Schmelzpunkt 74—76° getrocknet.

Beispiel 3

74,0 g cis-Akonitsäure-anhydrid wurden in 200 ml 100 g Eis enthaltendem Wasser gelöst. Eine Lösung von 46,25 g Natriumhydroxyd in 100 ml Wasser wurde unter Rühren zugesetzt. Die Temperatur wurde unterhalb 20° gehalten. Dann wurden nacheinander 15,25 g Natrium-wolframat-dihydrat und 55,5 ml 30%iges Wasserstoffperoxyd dem Gemisch zugesetzt. Die gerührte Lösung wurde auf 23° erhitzt. Die externe Wärmequelle wurde entfernt. Durch die Reaktionswärme stieg die Temperatur innert 25 Minuten auf 51,5° und fing dann an abzunehmen. Nach 30—40 Minuten Rühren wurde das Gemisch mit 150 ml konzentrierter Salzsäure und 150 g Natriumchlorid behandelt und 15 Minuten auf 75° erhitzt. Auf diese Weise wurde das Zwischenprodukt (±)-erythro-Epoxyaconitsäure in die (±)-erythro-Chlorcitronensäure übergeführt. Nach Abkühlen des Gemisches auf Raumtemperatur wurden 1,3 g Natriumbisulfit zur Zerstörung von überschüssigem Wasserstoffperoxyd zugesetzt. Die Lösung wurde dann kontinuierlich mit Aether extrahiert.

Der erste Extrakt wurde nach 21 Stunden aufgesammelt, getrocknet und zu 73,0 g roher Chlorcitronensäure konzentriert. Zweimalige Umkristallisierung aus Aethylacetat-Tetrachlorkohlenstoff ergab praktisch reine (±)-erythro-Chlorcitronensäure, Schmelzpunkt 162—164°.

Ein zweiter Extrakt ergab zusätzliche 18,5 g Säure, Schmelzpunkt 163—165°.

Beispiel 4

Eine Lösung von Trinatrium-trans-aconitat, hergestellt aus 58,0 g trans-Aconitsäure und 40 g Natriumhydroxyd in 300 ml Wasser wurde auf 5° abgekühlt und wie im Beispiel 1 chloriert. Die entstandene Lösung von Dinatriumchlorcitronensäure-$\beta$-lacton wurde von überschüssigem Chlor gereinigt und dann mit 60 ml 12N Salzsäure behandelt. Das Gemisch wurde mit Aethylacetat extrahiert und die Extrakte wurden vereinigt und getrocknet. Die Aethylacetatlösung wurde konzentriert und dann mit Tetrachlorkohlenstoff verdünnt. Das resultierende kristalline Material wurde filtriert und ergab 41,5 g reine (±)-threo-Chlorcitronensäure-$\beta$-lacton, Schmelzpunkt 162—164°.

Beispiel 5

30 g (±)-erythro-Chlorcitronensäure wurden in 175 ml eines Methanol-Wasser-Gemisches (49:1) gelöst. Die Lösung wurde auf 15° abgekühlt und mit 39,5 g (—)-p-Nitro-$\alpha$-methylbenzylamin in 75 ml des gleichen Methanol-Wasser-Gemisches versetzt. Das Gemisch wurde bei Raumtemperatur während 18 Stunden gerührt. Die Feststoffe wurden filtriert und mit Aethanol und Aether gewaschen, wobei man 24,0 g partiell aufgetrenntes (+)-erythro-Chlorcitronensäure-bis-(—)-p-nitro-$\alpha$-methylbenzylaminsalz erhielt.

Das unreine Salze wurde dann auf folgende Weise gespalten. Gasförmiger Chlorwasserstoff wurde 30 Minuten durch eine gerührte Suspension von 27,1 g Salz in Aether geleitet. Das entstandene feste (—)-p-Nitro-$\alpha$-methylbenzylamin-hydrochlorid (19,9 g, Schmelzpunkt 247—249°) wurde abfiltriert und das Filtrat zu 10,9 g partiell aufgetrennter (65% optische Reinheit) (+)-erythro-Chlorcitronensäure in Form eines Oels konzentriert.

Das (—)-p-Nitro-$\alpha$-methylbenzylamin-hydrochlorid wurde zwischen Dichlormethan und 1N Natriumhydroxyd verteilt. Das auf diese Weise erhaltene Amin (15,6 g) wurde in 40 ml Methanol-Wasser (49:1) einer Lösung von roher (+)-erythro-Chlorcitronensäure in 40 ml Methanol-Wasser (49:1) zugesetzt. Nach Rühren erhielt man 18,1 g angereichertes bis-Amin-chlorcitrat.

Das Salz wurde nochmals mit ätherischer Salzsäure gespalten und wie weiter oben beschrieben rückgebildet, wobei man 14,4 g bis-Amin-chlorcitrat erhielt. Die aus dem letzten Salz gewonnene (+)-erythro-Chlorcitronensäure wurde aus Aethylacetat-Tetrachlorkohlenstoff zu 4,3 g (29% chemische Ausbeute) Produkt mit 85%iger optischer Reinheit umkristallisiert.

Die Mutterlaugen der Lösung des partiell aufgetrennten (+)-erythro-Chlorcitronensäure-bis-(—)-p-nitro-$\alpha$-methylbenzylaminsalz wurden mit 13 ml konzentrierter Salzsäure behandelt und eingedampft. Aether wurde dem Rückstand zugesetzt, das Gemisch gerührt und der Niederschlag aufgesammelt, wobei man 29,2 g (—)-p-Nitro-$\alpha$-methylbenzylamin-hydrochlorid erhielt.

Das Filtrat wurde zur Trockene eingeengt und der an (—)-erythro-Chlorcitronensäure angereicherte Rückstand wurde in 90 ml Methanol-Wasser (49:1) gelöst. Eine Lösung von 25,3 g (+)-p-Nitro-$\alpha$-methylbenzylamin und 50 ml Methanol-Wasser (49:1) wurden zugesetzt. Das Gemisch wurde 20 Stunden gerührt und der Niederschlag aufgesammelt, wobei man 15,0 g bis-(+)-p-Nitro-$\alpha$-methylbenzylaminsalz angereichert an(—)-erythro-Chlorcitronensäure erhielt. Das Salz wurde in 185 ml

Aether suspendiert und Salzsäure wurde der Suspension zugesetzt. Das ausgefallene (+)-p-Nitro-$\alpha$-methylbenzylamin-hydrochlorid (10,7 g) wurde filtriert. Das Filtrat wurde zur Trockene eingedampft und der Rückstand in 40 ml Methanol-Wasser (49:1) gelöst. Der Methanol-Wasser-Lösung wurde eine Lösung von 8,9 g (+)-p-Nitro-$\alpha$-methylbenzylamin und 15 ml Methanol-Wasser (49:1) zugesetzt und die Lösung wurde 2,5 Stunden gerührt. Der Niederschlag (11,75 g) angereichert an (—)-erythro-Chlorcitronensäure-bis-(+)-p-nitro-$\alpha$-methylbenzylaminsalz wurde in Aether suspendiert und die Suspension wurde mit Salzsäure gesättigt. Das ausgefallene (+)-p-Nitro-$\alpha$-methylbenzylamin-hydrochlorid (8,09 g) wurde aufgesammelt und das Filtrat wurde konzentriert. Nach Umkristallisation des Rückstandes aus Aethylacetat-Tetrachlorkohlenstoff erhielt man 3,9 g (—)-erythro-Chlorcitronensäure (85% optische Reinheit).

Beispiel 6

105 g (+)-threo-Epoxyaconitsäure-monohydrat wurden in 150 ml 43 ml konzentrierte Salzsäure enthaltendem Wasser gelöst. 50 g Natriumchlorid wurden der gerührten Lösung zugesetzt und das Gemisch 12 Stunden bei 70° erhitzt. Die Lösung wurde zur Trockene eingeengt und der Rückstand mit 400 ml Aethylacetat vermischt. Das Gemisch wurde filtriert und das Filtrat wurde entfärbt, getrocknet und konzentriert. Der Rückstand wurde in 250 ml Aethylacetat gelöst und die Lösung wurde mit Tetrachlorkohlenstoff behandelt. Das Gemisch wurde mehrere Stunden gerührt und dann abgekühlt. Die Feststoffe wurden abfiltriert und man erhielt 68,5 g (—)-threo-Chlorcitronensäure, Schmelzpunkt 138—140°, $[\alpha]_d^{25}$ —6,60° (c, 2,0, $H_2O$). Aus den Mutterlaugen erhielt man 20,2 g zusätzliches Material.

Beispiel 7

(—)-threo-Epoxyaconitsäure-monohydrat (105 g) wurde in 150 ml 43,0 ml konzentrierte Salzsäure enthaltendem Wasser gelöst. Der gerührten Lösung wurden 50 g Natriumchlorid zugesetzt und das Gemisch wurde 12 Stunden bei 70° erhitzt. Durch Aufarbeiten des Gemisches wie in Beispiel 6 erhielt man (+)-threo-Chlorcitronensäure in zwei Anteilen:

Anteil 1: Schmelzpunkt 138—140°; $[\alpha]_D^{25}$ +6,65° (c, 2,0, $H_2O$); 55,2 g

Anteil 2: Schmelzpunkt 138—140°; $[\alpha]_D^{25}$ +6,55° (c, 2,0, $H_2O$); 23,0 g.

Durch Umkristallisation des Feststoffes aus Aethylacetat-Tetrachlorkohlenstoff erhielt man analytisch reines Material, Schmelzpunkt 140,5—142°; $[\alpha]_D^{25}$ +6,9° (c, 2,0, $H_2O$).

Beispiel 8

Eine Lösung von 8,1 g (+)-erythro-Epoxyaconitsäure in 43 ml 15 g Natriumchlorid enthaltender Salzsäure wurde 25 Minuten bei 78° und 20 Minuten bei 80° erhitzt. Durch Abdampfen des Lösungsmittels erhielt man einen Rückstand bestehend aus roher (—)-erythro-Chlorcitronensäure und Natriumchlorid. Das organische Material wurde in Glym gelöst und die Lösung wurde filtriert, getrocknet und konzentriert. Nach Kristallisation des Produktes aus Aethylacetat-Tetrachlorkohlenstoff erhielt man 7,4 g praktisch reine (—)-erythro-Chlorcitronensäure. Nach Umkristallisation erhielt man 5,4 g reine Säure, Schmelzpunkt 133,5—135°; $[\alpha]_D^{25}$ —2,2° (c, 2,0, $H_2O$).

Beispiel 9

(—)-erythro-Epoxyaconitsäure (9,5 g) wurde in 7,6 g (+)-erythro-Chlorcitronensäure (Schmelzpunkt 132—134°) in zu Beispiel 8 analoger Weise übergeführt. Umkristallisation der erhaltenen Chlorcitronensäure ergab 5,3 g analytisch reines Material, Schmelzpunkt 133,5—135°; $[\alpha]_D^{25}$ +2,2° (c, 2,0, $H_2O$).

Beispiel 10

87,0 g trans-Aconitsäure wurden portionenweise einer Lösung von 70,0 g Natriumhydroxyd in 200 ml Wasser zugesetzt. Das Gemisch wurde abgekühlt und wie in Beispiel 1 chloriert. Die resultierende Lösung von (±)-threo-Chlorcitronensäure-$\beta$-lacton-dinatriumsalz (von überschüssigem Chlor bereinigt) wurde auf —10° abgekühlt und mit 40,0 g Natriumhydroxyd behandelt. Die Lösung wurde gerührt und das Gemisch durch Abkühlen unterhalb 20° gehalten. Das Gemisch wurde 20 Minuten bei 20° gerührt. Dann wurden unter Abkühlen 42 ml konzentrierte Schwefelsäure zugesetzt. Die Lösung wurde mit Diäthyläther extrahiert. Der Aetherextrakt wurde getrocknet und konzentriert. Der entstandene Feststoff wurde aus Aethylacetat-Tetrachlorkohlenstoff zu 67,8 g (±)-threo-Epoxyaconitsäure, Schmelzpunkt 169—172° kristallisiert. Ein zweiter Anteil von 8,85 g, Schmelzpunkt 167—170° wurde von den Mutterlaugen erhalten.

Beispiel 11

Tabletten folgender Zusammensetzung wurden hergestellt:

| | |
|---|---|
| (—)-threo-Chlorcitronensäure | 50 mg |
| Polyvinylpyrrolidon | 2 mg |
| Mikrokristalline Cellulose | 10 mg |
| Siliziumdioxyd | 1 mg |
| Magnesiumstearat | 1 mg |

**Patentansprüche für die Vertragsstaaten: BE CH DE FR GB IT LU NL SE**

1. Monochlorcitronensäurederivate der Formel

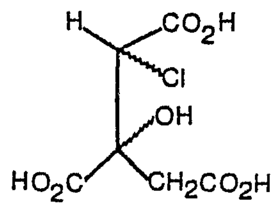

Ia

und entsprechende threo-$\beta$-Lactone der Formel

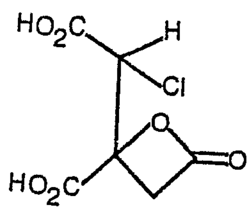

Ib

und pharmazeutisch verwendbare Salze dieser Verbindungen.

2. Verbindungen nach Anspruch 1 der Formel Ia und pharmazeutisch verwendbare Salze davon.
3. ($\pm$)-erythro-Chlorcitronensäure.
4. (+)-erythro-Chlorcitronensäure.
5. (—)-erythro-Chlorcitronensäure.
6. ($\pm$)-threo-Chlorcitronensäure.
7. (+)-threo-Chlorcitronensäure.
8. (—)-threo-Chlorcitronensäure.
9. Verbindungen nach Anspruch 1 der Formel Ib und pharmazeutisch verwendbare Salze davon.
10. Eine Verbindung der Gruppe der folgenden: ($\pm$)-threo-Chlorcitronensäure-$\beta$-lacton, (+)-threo-Chlorcitronensäure-$\beta$-lacton und (—)-threo-Chlorcitronensäure-$\beta$-lacton.
11. Eine Verbindung nach einem der Ansprüche 1—10 als pharmazeutischer Wirkstoff.
12. Eine Verbindung nach einem der Ansprüche 1—10 als Anorektikum und Mittel gegen Fettsucht.
13. (—)-threo-Chlorcitronensäure als pharmazeutischer Wirkstoff.
14. (—)-threo-Chlorcitronensäure als Anorektikum und Mittel gegen Fettsucht.
15. Verfahren zur Herstellung von Monochlorcitronensäurederivaten der Formeln Ia und Ib in Anspruch 1 sowie der pharmazeutisch verwendbaren Salze davon, dadurch gekennzeichnet, dass man a) zur Herstellung eines ($\pm$)-threo-Lactons der Formel Ib, eine wässrige Lösung eines Trialkalimetall- oder Trierdalkalimetall-cis- oder -trans-aconitats mit Chlor oder unterchloriger Säure umsetzt und das erhaltene Salz des ($\pm$)-threo-Chlorcitronensäure-$\beta$-lactons der Formel

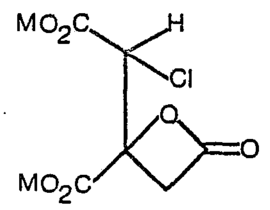

Ib 1

worin M ein Alkali- oder Erdalkalimetall ist, mit einer Säure umsetzt,
b) zur Herstellung der (±)-threo-Chlorcitronensäure der Formel

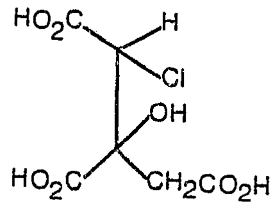

Ia 1

eine Verbindung der Formel Ib oder Ib 1 hydrolysiert,
c) zur Herstellung einer Verbindung der Formel Ia Epoxyakonitsäure mit einem Alkali- oder Erdalkalimetallchlorid in einem wässrigen Lösungsmittel in Gegenwart einer Säure spaltet,
d) zur Herstellung von (±)-erythro-Chlorcitronensäure der Formel

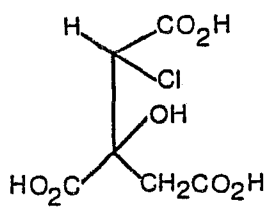

Ia 2

einer Verbindung der Formel

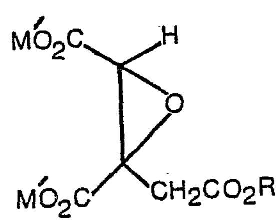

II

worin M′ ein Alkalimetall und R Wasserstoff oder M′ darstellt,
mit einem Alkalimetallchlorid in einem wässrigen Lösungsmittel in Gegenwart einer Säure spaltet,
e) gewünschtenfalls ein erhaltenes (±)-threo-Citronensäurederivat der Formel Ia oder Ib oder (±)-erythro-Citronensäurederivat der Formel Ia in die optisch aktiven Antipoden auftrennt und das erwünschte Antipode isoliert,
f) gewünschtenfalls eine erhaltene Verbindung der Formel Ia oder Ib in Form eines pharmazeutisch verwendbaren Salzes isoliert.

16. Verfahren nach Anspruch 15, dadurch gekennzeichnet, dass man (±)-threo-Epoxyaconitsäure in (±)-threo-Chlorcitronensäure überführt.

17. Verfahren nach Anspruch 15, dadurch gekennzeichnet, dass man (±)-erythro-Epoxyaconitsäure in (±)-erythro-Chlorcitronensäure überführt.

18. Verfahren nach Anspruch 15, dadurch gekennzeichnet, dass man (+)threo-Epoxyaconitsäure in (—)-threo-Chlorcitronensäure überführt.

19. Verfahren nach Anspruch 15, dadurch gekennzeichnet, dass man (—)-threo-Epoxyaconitsäure in (+)-threo-Chlorcitronensäure überführt.

20. Verfahren nach Anspruch 15, dadurch gekennzeichnet, dass man (—)-erythro-Epoxyaconitsäure in (+)-erythro-Chlorcitronensäure überführt.

21. Verfahren nach Anspruch 15, dadurch gekennzeichnet, dass man (+)-erythro-Epoxyaconitsäure in (—)-erythro-Chlorcitronensäure überführt.

22. Arzneimittel enthaltend eine Verbindung der Formel Ia oder Ib nach einem der Ansprüche 1—10 oder ein pharmazeutisch verwendbares Salz davon.

23. Anorektikum und Mittel gegen Fettsucht enthaltend eine Verbindung der Formel Ia oder Ib nach einem der Ansprüche 1—10 oder ein pharmazeutisch verwendbares Salz davon.

24. Arzneimittel enthaltend (—)-threo-Chlorcitronensäure.

25. Anorektikum und Mittel gegen Fettsucht enthaltend (—)-threo-Chlorcitronensäure.

**Patentansprüche für den Vertragsstaat: AT**

1. Verfahren zur Herstellung von Monochlorcitronensäurederivaten der Formel

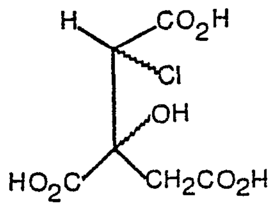

Ia

und der entsprechenden threo-β-Lactone der Formel

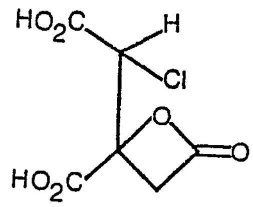

Ib

sowie der pharmazeutisch verwendbaren Salze davon, dadurch gekennzeichnet, dass man
a) zur Herstellung eines (±)-threo-Lactons der Formel Ib, eine wässrige Lösung eines Trialkalimetall- oder Trierdalkalimetall-cis- oder -trans-aconitats mit Chlor oder unterchloriger Säure umsetzt und das erhaltene Salz des (±)-threo-Chlorcitronensäure-β-lactons der Formel

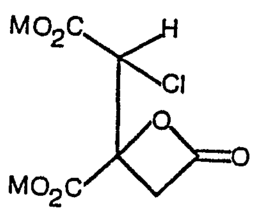

Ib 1

worin M ein Alkali- oder Erdalkalimetall ist, mit einer Säure umsetzt,
b) zur Herstellung der (±)-threo-Chlorcitronensäure der Formel

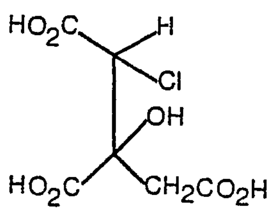

Ia 1

eine Verbindung der Formel Ib oder Ib 1 hydrolysiert,
c) zur Herstellung einer Verbindung der Formel Ia Epoxyaconitsäure mit einem Alkali- oder Erdalkalimetallchlorid in einem wässrigen Lösungsmittel in Gegenwart einer Säure spaltet,
d) zur Herstellung von (±)-erythro-Chlorcitronensäure der Formel

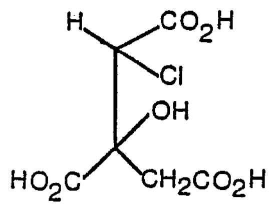

Ia 2

einer Verbindung der Formel

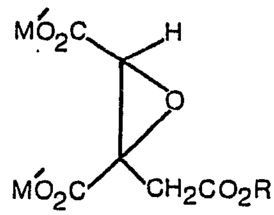

II

worin M' ein Alkalimetall und R Wasserstoff oder M' darstellt, mit einem Alkalimetallchlorid in einem wässrigen Lösungsmittel in Gegenwart einer Säure spaltet,

e) gewünschtenfalls ein erhaltenes (±)-threo-Citronensäurederivat der Formel Ia oder Ib oder (±)-erythro-Citronensäurederivat der Formel Ia in die optisch aktiven Antipoden auftrennt und das erwünschte Antipode isoliert,

f) gewünschtenfalls eine erhaltene Verbindung der Formel Ia oder Ib in Form eines pharmazeutisch verwendbaren Salzes isoliert.

2. Verfahren nach Anspruch 1, dadurch gekennzeichnet, dass man (±)-threo-Epoxyaconitsäure in (±)-threo-Chlorcitronensäure überführt.

3. Verfahren nach Anspruch 1, dadurch gekennzeichnet, dass man (±)-erythro-Epoxyaconitsäure in (±)-erythro-Chlorcitronensäure überführt.

4. Verfahren nach Anspruch 1, dadurch gekennzeichnet, dass man (+)-threo-Epoxyaconitsäure in (—)-threo-Chlor-citronensäure überführt.

5. Verfahren nach Anspruch 1, dadurch gekennzeichnet, dass man (—)-threo-Epoxyaconitsäure in (+)-threo-Chlorcitronensäure überführt.

6. Verfahren nach Anspruch 1, dadurch gekennzeichnet, dass man (—)-erythro-Epoxyaconitsäure in (+)-erythro-Chlorcitronensäure überführt.

7. Verfahren nach Anspruch 1, dadurch gekennzeichnet, dass man (+)-erythro-Epoxyaconitsäure in (—)-erythro-Chlorcitronensäure überführt.

**Claims for the Contracting States: BE CH DE FR GB IT LU NL SE**

1. Monochlorocitric acid derivatives of the formula

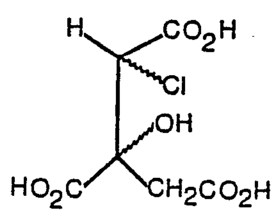

Ia

and the corresponding threo-β-lactones of the formula

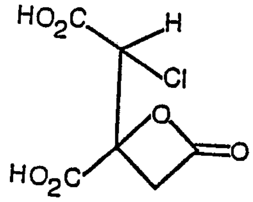

Ib

and pharmaceutically usable salts of these compounds.

2. Compounds according to claim 1 of formula Ia and pharmaceutically usable salts thereof.

3. (±)-Erythro-chlorocitric acid.

4. (+)-Erythro-chlorocitric acid.

5. (—)-Erythro-chlorocitric acid.

6. (±)-Threo-chlorocitric acid.

7. (+)-Threo-chlorocitric acid.

8. (—)-Threo-chlorocitric acid.

9. Compounds according to claim 1 of formula Ib and pharmaceutically usable salts thereof.

10. A compound of the group of the following: (±)-threo-chlorocitric acid β-lactone, (+)-threo-chlorocitric acid β-lactone and (—)-threo-chlorocitric acid β-lactone.

11. A compound according to any one of claims 1—10 as a pharmaceutically active substance.

12. A compound according to any one of claims 1—10 as an anorectic and agent against obesity.

13. (—)-Threo-chlorocitric acid as a pharmaceutically active substance.

14. (—)-Threo-chlorocitric acid as an anorectic anc agent against obesity.

15. Process for the manufacture of monochlorocitric acid derivatives of formula Ia and Ib in claim 1 as well as the pharmaceutically usable salts thereof, characterized by

a) for the manufacture of a (±)-threo-lactone of formula Ib, reacting an aqueous solution of a trialkali metal or trialkaline earth metal cis- or trans-aconitate with chlorine or hypochlorous acid and reacting the resulting salt of (±)-threo-chlorocitric acid β-lactone of the formula

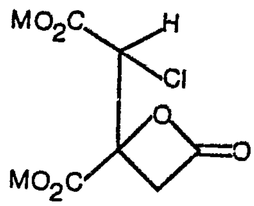

Ib 1

wherein M is an alkali metal or alkaline earth metal, with an acid,

b) for the manufacture of (±)-threo-chlorocitric acid of the formula

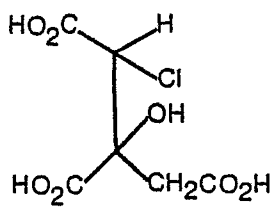

Ia 1,

hydrolyzing a compound of formula Ib or Ib 1,

c) for the manufacture of a compound of formula Ia, cleaving epoxyaconitic acid with an alkali metal or alkaline earth metal chloride in an aqueous solvent in the presence of an acid,

d) for the manufacture of (±-erythro-chlorocitric acid of the formula

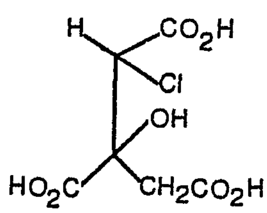

Ia2,

cleaving a compound of the formula

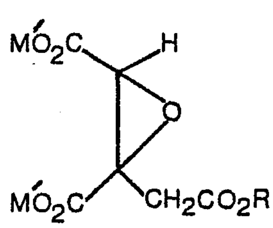

II

wherein M' represents an alkali metal and R represents hydrogen or M', with an alkali metal chloride in an aqueous solvent in the presence of an acid,
e) if desired, resolving a resulting (±)-threo-citric acid derivative of formula Ia or Ib or (±)-erythro-citric acid derivative of formula Ia into the optically active antipodes and isolating the desired antipode,
f) if desired, isolating a resulting compound of formula Ia or Ib in the form of a pharmaceutically usable salt.

16. Process according to claim 15, characterized in that (±)-threo-epoxyaconitic acid is converted into (±)-threo-chlorocitric acid.

17. Process according to claim 15, characterized in that (±)-erythro-epoxyaconitic acid is converted into (±)-erythro-chlorocitric acid.

18. Process according to claim 15, characterized in that (+)-threo-epoxyaconitic is converted into (—)-threo-chlorocitric acid.

19. Process according to claim 15, characterized in that (—)-threo-epoxyaconitic acid is converted into (+)-threo-chlorocitric acid.

20. Process according to claim 15, characterized in that (—)-erythro-epoxyaconitic acid is converted into (+)-erythro-chlorocitric acid.

21. Process according to claim 15, characterized in that (+)-erythro-epoxyaconitic acid is converted into (—)-erythro-chlorocitric acid.

22. Medicament containing a compound of formula Ia or Ib according to any one of claims 1—10 or a pharmaceutically usable salt thereof.

23. Anorectic and agent against obesity containing a compound of formula Ia or Ib according to any one of claims 1—10 or a pharmaceutically usable salt thereof.

24. Medicament containing (—)-threo-chlorocitric acid.

25. Anorectic and agent against obesity containing (—)-threo-chlorocitric acid.

**Claims for the Contracting State: AT**

1. Process for the manufacture of monochlorocitric acid derivatives of the formula

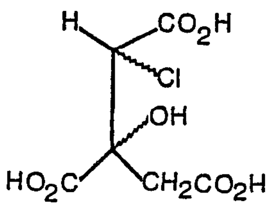

Ia

and the corresponding threo-β-lactones of the formula

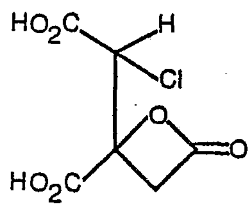

I

as well as the pharmaceutically usable salts thereof, characterized by
a) for the manufacture of a (±)-threo-lactone of formula Ib, reacting an aqueous solution of a trialkali metal or trialkaline earth metal cis- or trans-aconitate with chlorine or hypochlorous acid and reacting the resulting salt of (±)-threo-chlorocitric acid β-lactone of the formula

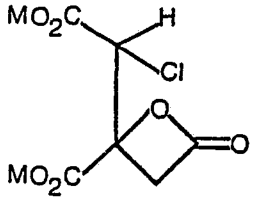

Ib 1

wherein M is an alkali metal or alkaline earth metal, with an acid,

b) for the manufacture of (±)-threo-chlorocitric acid of the formula

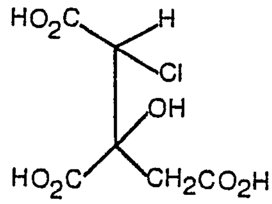

Ia 1,

hydrolyzing a compound of formula Ib or Ib 1,
c) for the manufacture of a compound of formula Ia, cleaving epoxyaconitic acid with an alkali metal or alkaline earth metal chloride in an aqueous solvent in the presence of an acid,
d) for the manufacture of (±)-erythro-chlorocitric acid of the formula

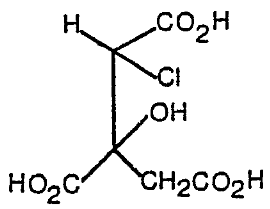

Ia 2,

cleaving a compound of the formula

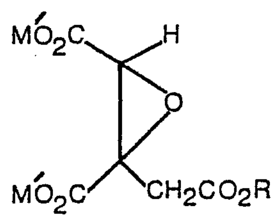

II

wherein M′ represents an alkali metal and R represents hydrogen or M′,
with an alkali metal chloride in an aqueous solvent in the presence of an acid,
e) if desired, resolving a resulting (±)-threo-citric acid derivative of formula Ia or Ib or (±)-erythro-citric acid derivative of formula Ia into the optically active antipodes and isolating the desired antipode,
f) if desired, isolating a resulting compound of formula Ia or Ib in the form of a pharmaceutically usable salt.

2. Process according to claim 1, characterized in that (±)-threo-epoxyaconitic acid is converted into (±)-threo-chlorocitric acid.

3. Process according to claim 1, characterized in that (±)-erythro-epoxyaconitic acid is converted into (±)-erythro-chlorocitric acid.

4. Process according to claim 1, characterized in that (+)-threo-epoxyaconitic acid is converted into (—)-threo-chlorocitric acid.

5. Process according to claim 1, characterized in that (—)-threo-epoxyaconitic acid is converted into (+)-threo-chlorocitric acid.

6. Process according to claim 1, characterized in that (—)-erythro-epoxyaconitic acid is converted into (+)-erythro-chlorocitric acid.

7. Process according to claim 1, characterized in that (+)-erythro-epoxyaconitic acid is converted into (—)-erythro-chlorocitric acid.

**Revendications pour les Etats contractants: BE CH DE FR GB IT LU NL SE**

1. Dérivés de l'acide monochlorocitrique de formule

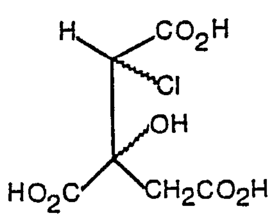

Ia

et thréo-β-lactones correspondantes de formule

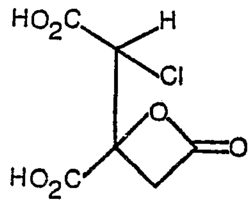

Ib

et sels de ces composés utilisables en pharmacie.

2. Composés selon la revendication 1, de formule Ia et leurs sels utilisables en pharmacie.
3. L'acide (±)-érythro-chlorocitrique.
4. L'acide (+)-érythro-chlorocitrique.
5. L'acide (—)-érythro-chlorocitrique.
6. L'acide (±)-thréo-chlorocitrique.
7. L'acide (+)-thréo-chlorocitrique.
8. L'acide (—)-thréo-chlorocitrique.
9. Composés selon la revendication 1, de formule Ib et leurs sels utilisables en pharmacie.
10. Un composé du groupe des suivants: la β-lactone de l'acide (±)-thréo-chlorocitrique, la β-lactone de l'acide (+)-thréo-chlorocitrique et la β-lactone de l'acide (—)-thréo-chlorocitrique.
11. Un composé selon l'une des revendications 1 à 10, en tant que substance active pharmaceutique.
12. Un composé selon l'une des revendications 1 à 10, en tant qu'anorexique et agent contre l'adiposité.
13. L'acide (—)-thréo-chlorocitrique en tant que substance active pharmaceutique.
14. L'acide (—)-thréo-chlorocitrique en tant qu'anorexique et agent contre l'adiposité.
15. Procédé de préparation des dérivés d'acides monochlorocitriques de formules Ia ou Ib de la revendication 1,

et de leurs sels utilisables en pharmacie, caractérisé en ce que:

a) pour la préparation d'une (±)-thréo-lactone de formule Ib, on fait réagir une solution aqueuse d'un cis- ou trans-aconitate de tri-métal alcalin ou de tri-métal alcalino-terreux avec le chlore ou l'acide hypochloreux et on fait réagir le sel de la β-lactone de l'acide (±)-thréo-chlorocitrique obtenu, de formule

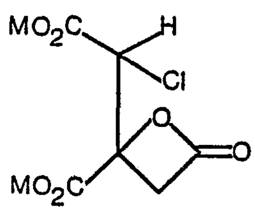

Ib 1

dans laquelle M représente un métal alcalin ou alcalinoterreux, avec un acide,

b) pour la préparation de l'acide (±)-thréo-chlorocitrique de formule

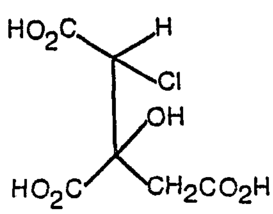

IA 1

on hydrolyse un composé de formule Ib ou Ib 1,

c) pour la préparation d'un composé de formule Ia, on scinde l'acide époxy-aconitique à l'aide d'un chlorure de métal alcalin ou alcalino-terreux dans un solvant aqueux en présence d'un acide,

d) pour la préparation de l'acide (±)-érythro-chlorocitrique de formule

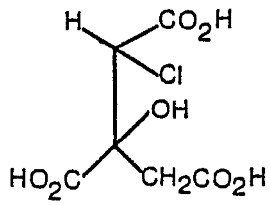

Ia 2

on scinde un composé de formule

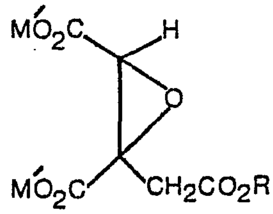

II

dans laquelle M' représente un métal alcalin et R l'hydrogène ou M',
à l'aide d'un chlorure de métal alcalin dans un solvant aqueux en présence d'un acide,
e) si on le désire, on sépare un dérivé d'acide (±)-thréo-citrique de formules Ia ou Ib ou un dérivé d'acide (±)-érythro-citrique de formule Ia, ainsi obtenu, en les antipodes optiques, ou on isole l'antipode recherché,
f) si on le désire, on isole un composé de formules Ia ou Ib, ainsi obtenu, à l'état de sel utilisable en pharmacie.

16. Procédé selon la revendication 15, caractérisé en ce que l'on convertit l'acide (±)-thréo-époxyaconitique en l'acide (±)-thréo-chlorocitrique.

17. Procédé selon la revendication 15, caractérisé en ce que l'on convertit l'acide (±)-érythro-époxyaconitique en acide (±)-érythro-chlorocitrique.

18. Procédé selon la revendication 15, caractérisé en ce que l'on convertit l'acide (+)-thréo-époxyaconitique en l'acide (—)-thréo-chlorocitrique.

19. Procédé selon la revendication 15, caractérisé en ce que l'on convertit l'acide (—)-thréo-époxyaconitique en l'acide (+)-thréo-chlorocitrique.

20. Procédé selon la revendication 15, caractérisé en ce que l'on convertit l'acide (—)-érythro-époxyaconitique en l'acide (+)-érythro-chlorocitrique.

21. Procédé selon la revendication 15, caractérisé en ce que l'on convertit l'acide (+)-érythro-époxyaconitique en l'acide (—)-érythro-chlorocitrique.

22. Médicament contenant un composé de formule Ia ou Ib selon l'une des revendications 1 à 10 ou un sel d'un tel composé utilisable en pharmacie.

23. Anoréxique et agent contre l'adiposité, contenant un composé de formules Ia ou Ib selon l'une des revendications 1 à 10, ou un sel d'un tel composé utilisable en pharmacie.

24. Médicament contenant de l'acide (—)-thréo-chlorocitrique.

25. Anoréxique et agent contre l'adiposité, contenant l'acide (—)-thréo-chlorocitrique.

**Revendications pour l'Etat contractant: AT**

1. Procédé de préparation des dérivés de l'acide monochlorocitrique de formule

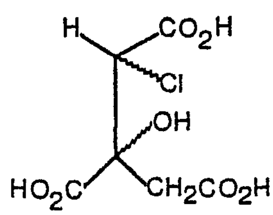

Ia

et des thréo-$\beta$-lactones correspondantes de formule

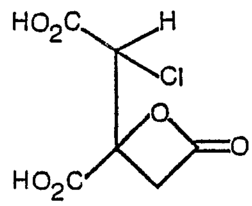

Ib

et de leurs sels utilisables en pharmacie, caractérisé en ce que:
a) pour la préparation d'une (±)-thréo-lactone de formule Ib, on fait réagir une solution aqueuse d'un cis- ou trans-aconitate de tris-métal alcalin ou de tris-métal alcalino terreux avec le chlore ou l'acide hypochloreux et on fait réagir le sel de la β-lactone de l'acide (±)-thréo-chlorocitrique ainsi obtenu, de formule

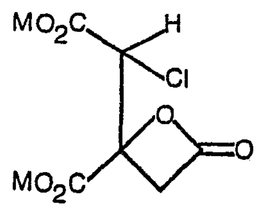

Ib 1

dans laquelle M représente un métal alcalin ou alcalino-terreux, avec un acide,
b) pour la préparation de l'acide (±)-thréo-chlorocitrique de formule

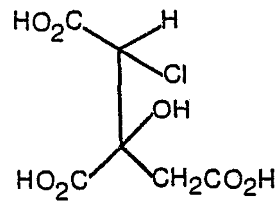

Ia 1

on hydrolyse un composé de formule Ib ou Ib 1,
c) pour la préparation d'un composé de formule Ia, on scinde l'acide époxy-aconitique à l'aide d'un chlorure de métal alcalin ou alcalino-terreux dans un solvant aqueux en présence d'un acide,
d) pour la préparation de l'acide (±)-érythro-chlorocitrique de formule

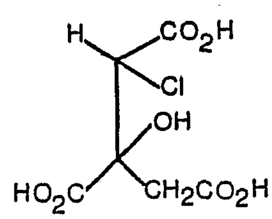

Ia 2

on scinde un composé de formule

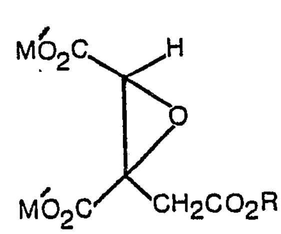

II

dans laquelle M′ représente un métal alcalin et R l'hydrogène ou M′,
à l'aide d'un chlorure de métal alcalin dans un solvant aqueux en présence d'un acide,
e) si on le désire, on sépare un dérivé de l'acide (±)-thréo-citrique de formules Ia ou Ib ou un dérivé de

l'acide (±)-érythro-citrique de formule Ia, ainsi obtenu, en les antipodes optiques et on isole l'antipode recherché,

f) si on le désire, on isole un composé de formules Ia ou Ib, ainsi obtenu, à l'état de sel utilisable en pharmacie.

2. Procédé selon la revendication 1, caractérisé en ce que l'on convertit l'acide (±)-thréo-époxy-aconitique en l'acide (±)-thréo-chlorocitrique.

3. Procédé selon la revendication 1, caractérisé en ce que l'on convertit l'acide (±)-érythro-époxy-aconitique en l'acide (±)-érythro-chlorocitrique.

4. Procédé selon la revendication 1, caractérisé en ce que l'on convertit l'acide (+)-thréo-époxy-aconitique en l'acide (—)-thréo-chlorocitrique.

5. Procédé selon la revendication 1, caractérisé en ce que l'on convertit l'acide (—)-thréo-époxy-aconitique en l'acide (+)-thréo-chlorocitrique.

6. Procédé selon la revendication 1, caractérisé en ce que l'on convertit l'acide (—)-érythro-époxy-aconitique en l'acide (+)-érythro-chlorocitrique.

7. Procédé selon la revendication 1, caractérisé en ce que l'on convertit l'acide (+)-érythro-époxy-aconitique en l'acide (—)-érythro-chlorocitrique.